# EUROPÄISCHE PATENTSCHRIFT

(11) **EP 0 907 648 B1**
(45) Veröffentlichungstag und Bekanntmachung des Hinweises auf die Patenterteilung: **06.03.2002**
(21) Anmeldenummer: 97928208.4
(22) Anmeldetag: 16.06.1997
(51) Int. Cl.: C07D 401/04, A61K 31/47

(54) **7-(3-VINYL-1,4-PIPERAZIN-1-YL)-SUBSTITUIERTE CHINOLONCARBONSÄUREN**
QUINOLONE CARBOXYLIC ACIDS SUBSTITUTED BY 7-(3-VINYL-1,4-PIPERAZINE-1-YL)
ACIDES QUINOLONE-CARBOXYLIQUES SUBSTITUES PAR 7-(3-VINYL-1,4-PIPERAZIN-1-YLE)

(30) Priorität: 28.06.1996 DE 19625988; 12.12.1996 DE 19651687
(43) Veröffentlichungstag der Anmeldung: 14.04.1999
(73) Patentinhaber: BAYER AG, 51368 Leverkusen (DE)
(72) Erfinder: HIMMLER, Thomas, D-51519 Odenthal (DE); JAETSCH, Thomas, D-50668 Köln (DE); HALLENBACH, Werner, D-40789 Monheim (DE); RAST, Hans-Georg, D-51465 Bergisch Gladbach (DE); WETZSTEIN, Heinz-Georg, D-51377 Leverkusen (DE); HEINEN, Ernst, D-54668 Echternacherbrück (DE); PIRRO, Franz, D-40764 Langenfeld (DE); SCHEER, Martin, D-42113 Wuppertal (DE); STEGEMANN, Michael, Kansas City, MO 64112 (US); STUPP, Hans-Peter, D-51379 Leverkusen (DE)
(86) Internationale Anmeldenummer: EP9703116
(87) Internationale Veröffentlichungsnummer: WO9800421

(56) Entgegenhaltungen:
- EP-A- 0 230 053

## Beschreibung

Die Erfindung betrifft neue 7-(3-Vinyl-1,4-piperazin-1-yl)-substituierte Chinoloncarbonsäuren, Verfahren zu ihrer Herstellung sowie diese enthaltende antibakterielle Mittel.

Aus EP-A 230 053 sind Chinoloncarbonsäuren mit einem 3-Vinyl-1,4-piperazin-Rest in der 7-Position bekannt geworden.

Es wurden neue Verbindungen der allgemeinen Formel (I) gefunden 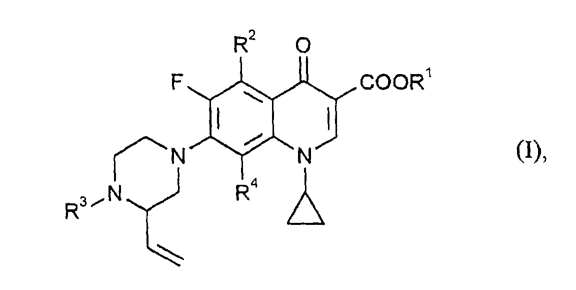 in welcher
- R¹: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- R²: für Wasserstoff oder Fluor steht, wenn gleichzeitig R⁴ ungleich Wasserstoff oder Fluor ist, oder für Amino, Methyl oder Vinyl steht,
- R³: für Wasserstoff, Benzyl, Alkyl mit 1 bis 3 Kohlenstoffatomen, Reste der Strukturen -CH=CH-COOR⁵, -CH₂CH₂-COOR⁵, -CH₂CH₂CN, -CH₂CH₂COCH₃, -CH₂COCH₃, in denen R⁵ für Methyl oder Ethyl steht, oder einen Rest der allgemeinen Struktur R⁶-(NH-CHR⁷-CO)ₙ- steht, in der R⁶ für Wasserstoff, Alkyl mit 1 bis 3 Kohlenstoffatomen oder den Rest -COO-tert.-Butyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht und n = 1 oder 2 ist, und
- R⁴: für Wasserstoff oder Fluor steht, wenn R² für Amino, Methyl oder Vinyl steht, oder für Chlor, Methoxy, Difluormethoxy, CN oder Ethinyl steht.

Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- und Guanidiniumsalze vorliegen.

Man erhält die Verbindungen der Formel (I), wenn man Verbindungen der Formel (II) 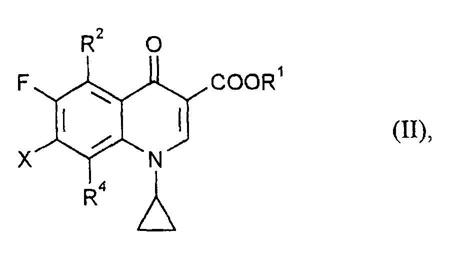 in welcher
- R¹, R² und R⁴: die oben angegebene Bedeutung haben und
- X: für Fluor oder Chlor steht,
mit Verbindungen der Formel (III) 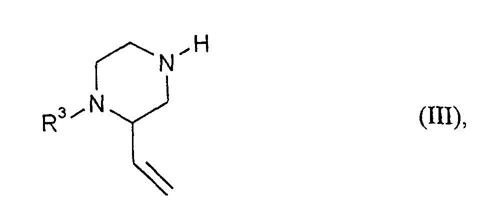 in welcher R³ die oben angegebene Bedeutung hat, gegebenenfalls in Gegenwart von Säurefängern umsetzt.

Die erfindungsgemäßen Verbindungen weisen im Vergleich zu bekannten Vertretern diesen Strukturtyps eine höhere antibakterielle Wirkung, insbesondere gegen E. coli, Staphylokokken, Streptokokken, Salmonellen und Mycoplasmen auf. Sie eignen sich daher als Wirkstoffe für die Human- und Veterinärmedizin, wobei zur Veterinärmedizin auch die Behandlung von Fischen zur Therapie oder zur Vorbeugung bakterieller Infektionen zu zählen ist.

Bevorzugt sind Verbindungen der Formel (I), in welcher
- R¹: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- R²: für Wasserstoff oder Fluor steht, wenn gleichzeitig R⁴ ungleich Wasserstoff oder Fluor ist, oder für Amino oder Methyl steht,
- R³: für Wasserstoff, Alkyl mit 1 bis 3 Kohlenstoffatomen, Reste der Strukturen -CH=CH-COOR⁵, -CH₂CH₂-COOR⁵, -CH₂CH₂CN, -CH₂COCH₃, R⁶-(NH-CHR⁷-CO)ₙ- steht, in der R⁶ für Wasserstoff, Alkyl mit 1 bis 3 Kohlenstoffatomen oder den Rest -COO-tert.-Butyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht und n = 1 oder 2 ist,
und
- R⁴: für Wasserstoff oder Fluor steht, wenn R² für Amino oder Methyl steht, oder für Chlor, Methoxy, Difluormethoxy oder CN steht,
und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind Verbindungen der Formel (I), in welcher
- R¹: für Wasserstoff, Methyl oder Ethyl steht,
- R²: für Wasserstoff oder Fluor steht, wenn gleichzeitig R⁴ ungleich Wasserstoff oder Fluor ist, oder für Amino steht,
- R³: für Wasserstoff, Methyl, Reste der Strukturen -CH=CH-COOR⁵, -CH₂CH₂-CN oder -CH₂COCH₃, in denen R⁵ für Methyl oder Ethyl steht, oder einen Rest der allgemeinen Struktur R⁶-(NH-CHR⁷-CO)ₙ- steht, in der R⁶ für Wasserstoff oder Methyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht, und n = 1 oder 2 ist,
und
- R⁴: für Wasserstoff oder Fluor steht, wenn R² für Amino steht, oder für Chlor, Methoxy, Difluormethoxy oder CN steht.

Als besonders bevorzugte Verbindungen der Formel (I) seien beispielsweise folgende genannt:
8-Chlor-1-cyclopropyl-6-fluor-1,4-dihydro-4-oxo-7-(3-vinyl-1,4-piperazin-1-yl)-3-chinolincarbonsäure,
5-Amino-1-cyclopropyl-6,8-difluor-1,4-dihydro-4-oxo-7-(3-vinyl-1,4-piperazin-1-yl)-3-chinolincarbonsäure,
1-Cyclopropyl-6-fluor-8-difluormethoxy-3,4-dihydro-4-oxo-7-(3-vinyl-1,4-piperazin- 1-yl)-3-chinolincarbonsäure,
8-Cyano-1-cyclopropyl-6-fluor-1,4-dihydro-4-oxo-7-(3-vinyl-1,4-piperazin-1-yl)-3-chinolincarbonsäure.

Verwendet man zur Herstellung von Verbindungen der Formel (I) beispielsweise 8-Chlor-1-cyclopropyl-6,7-difluor-1,4-dihydro-4-oxo-chinolin-3-carbonsäure und 2-Vinyl-1,4-piperazin, so kann der Reaktionsverlauf durch das folgende Formelschema wiedergegeben werden:

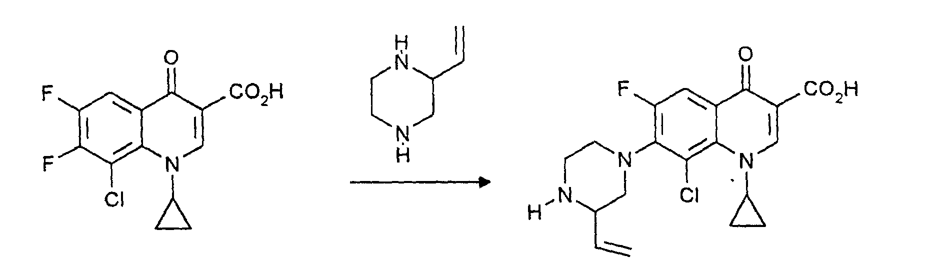

Verbindungen der Formel (I) können auch dadurch erhalten werden, daß man nach der Umsetzung einer Verbindung der Formel (II) mit 2-Vinyl-1,4-piperazin (R³ = H in Formel (III)) eine weitere Umsetzung des erhaltenen Produktes durchführt. So können Verbindungen der Formel (I) mit R³ gleich einem Rest -CH=CH-COOEt beispielsweise nach folgendem Formelschema erhalten werden:

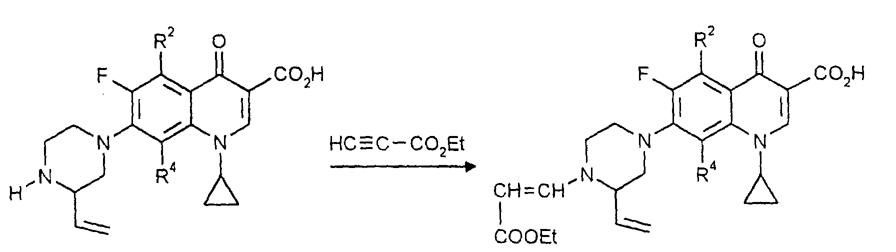

Als weiteres Beispiel sei die Umsetzung einer Verbindung der Formel (I) mit R³ = Wasserstoff zu einer Verbindung der Formel (I) mit R³ = Methyl genannt:

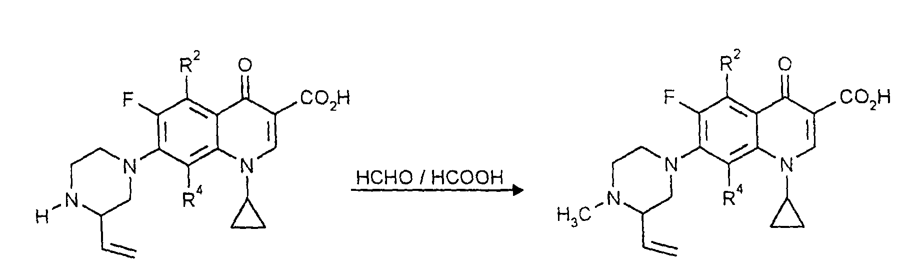

Die als Ausgangsverbindungen verwendeten Verbindungen der Formel (II) sind bekannt bzw. können nach bekannten Verfahren hergestellt werden.

Als Beispiele seien genannt:
8-Chlor-1-cyclopropyl-6,7-difluor-1,4-dihydro-4-oxo-3-chinolincarbonsäure,
5-Amino-1-cyclopropyl-6,7,8-trifluor-1,4-dihydro-4-oxo-3-chinolincarbonsäure,
1-Cyclopropyl-8-ethinyl-6,7-difluor-1,4-dihydro-4-oxo-3-chinolincarbonsäure,
7-Chlor-8-cyano-1-cyclopropyl-6-fluor-1,4-dihydro-4-oxo-3-chinolincarbonsäure,
1-Cyclopropyl-6,7-difluor-8-difluormethoxy-1,4-dihydro-4-oxo-3-chinolincarbonsäure.

Die als Ausgangsverbindungen verwendeten Piperazine der Formel (III) sind bekannt.

Als Beispiel sei 2-Vinyl-1,4-piperazin genannt.

Diese Verbindung wird in der EP-A 230 053 genannt.

Die Synthese kann beispielsweise durch Debenzylierung von 1-Benzyl-3-vinyl-piperazin oder 1,4-Dibenzyl-2-vinyl-piperazin erfolgen:

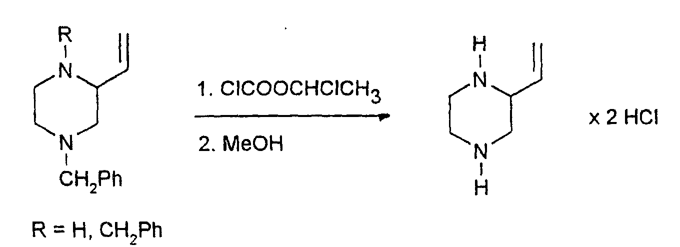

1-Benzyl-3-vinyl-piperazin und 1,4-Dibenzyl-2-vinyl-piperazin sind literaturbekannte Verbindungen.

Die Vinylpiperazine können sowohl als Racemate, als auch als enantiomerenreine Verbindungen eingesetzt werden.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykol-monomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 mol der Verbindung (II) 1 bis 15 mol, vorzugsweise 1 bis 6 mol der Verbindung (III) ein.

Freie Aminogruppen können während der Umsetzung durch eine geeignete Aminoschutzgruppe, z. B. durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise z.B. durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung vor allem auf resistente Keime und Mycoplasmen aus.

Gegenüber Bakterien, die gegenüber vergleichbaren Substanzen als weniger empfindlich eingestuft werden, insbesondere resistenten Staphylococcus aureus und E. E. coli zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektions- und orale verabreichbare Lösungen, Suspensionen und Emulsionen, ferner Pasten, Salben, Gele, Cremes, Lotions, Puder und Sprays genannt.

Die Wirkstoffe eignen sich bei günstiger Warmblütertoxizität bevorzugt zur Bekämpfung von bakteriellen Erkrankungen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säuretiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die minimalen Hemmkonzentrationen (MHK) der erfindungsgemäßen Verbindungen wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war. Die Erfassung der MHK-Werte für Mycoplasmen erfolgte mikroskopisch nach einer Inkubationszeit von 5 bis 7 Tagen.

In den nachstehenden Tabellen sind die MHK-Werte einiger der erfindungsgemäßen Verbindungen aufgeführt.

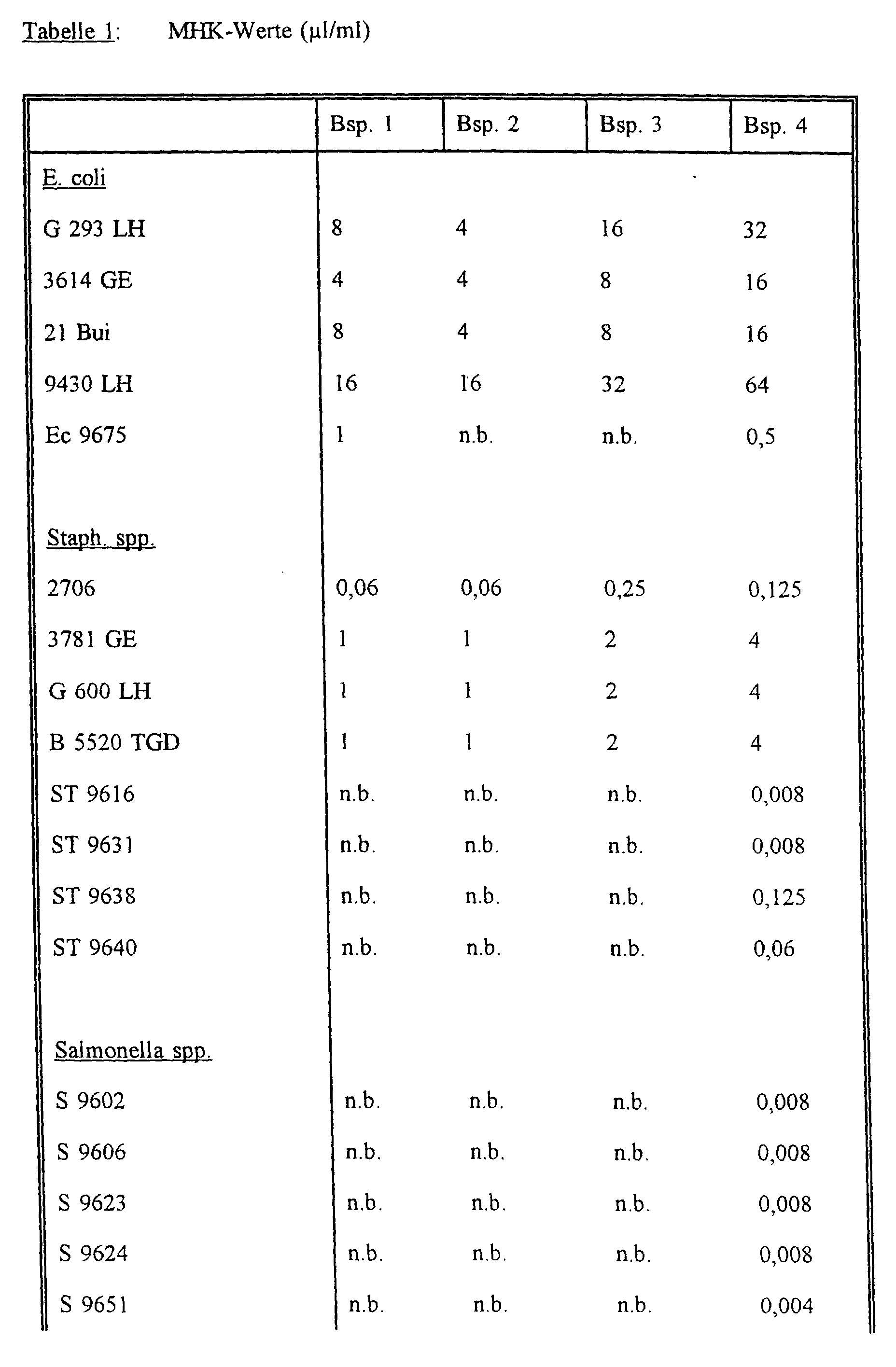

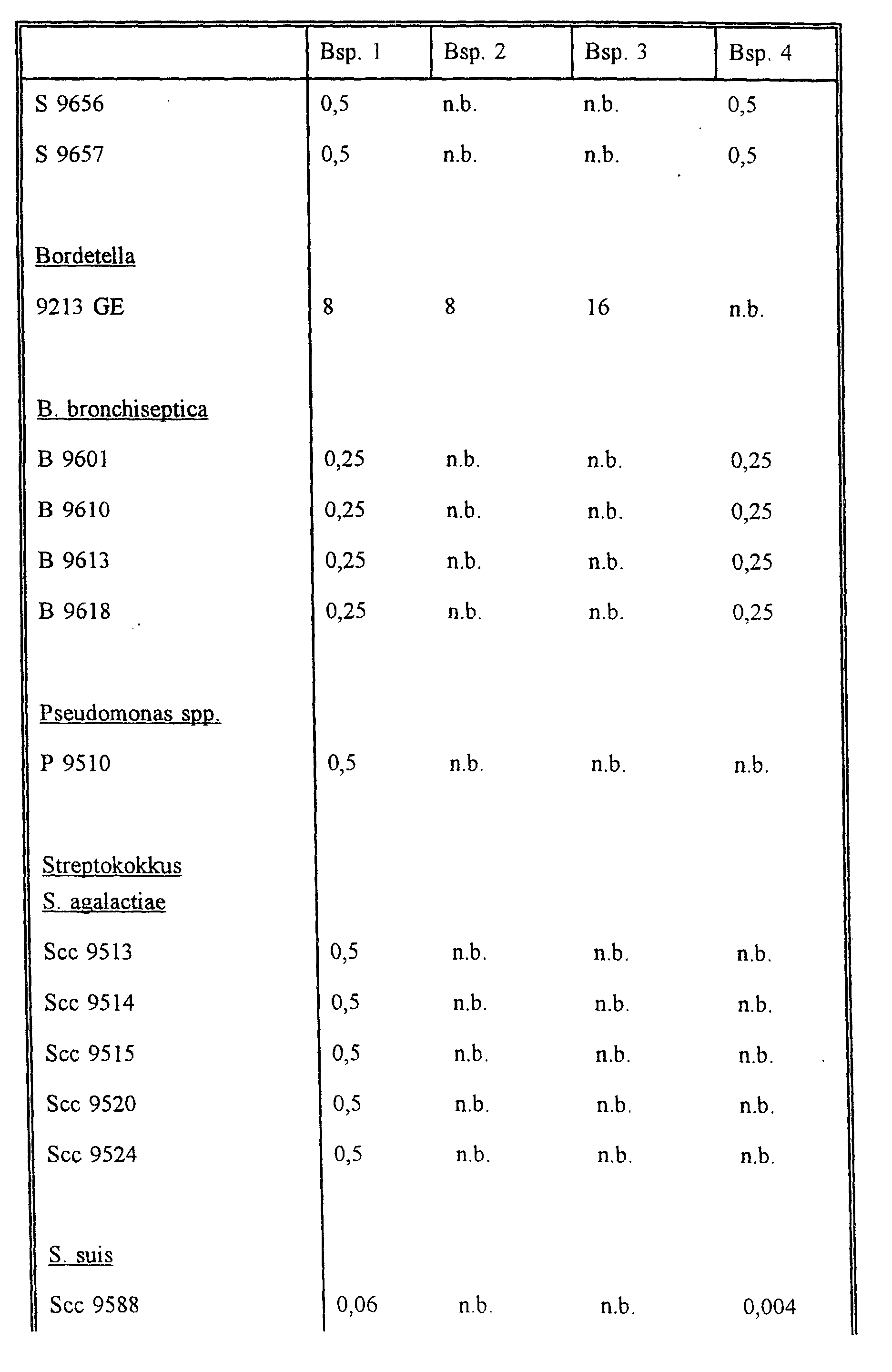

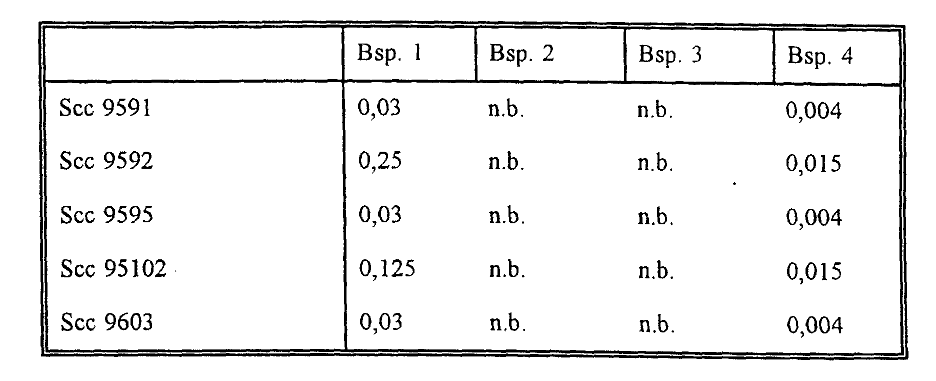

**Tabelle 2**

| MHK-Werte gegen Mycoplasmen (µg/ml) | | |
|---|---|---|
| | Bsp. 1 | Bsp4 |
| M. agalactiae PG 2 | 0,06 | 0,03 |
| M. bovis "Donetta" | 0,06 | 0,125 |
| M. bovirhinis PG 43 | 0,06 | 0,25 |
| M. gallisepticum PG 31 | 0,03 | 0,03 |
| M. iowae 695 | 0,03 | 0,06 |

Die antibakterielle Wirksamkeit der erfindungsgemäßen Verbindung in-vivo wurde folgendermaßen bestimmt. Zwei bis drei Wochen alte Hühner-Küken (Lohmann-Hybriden, Fa. Lohmann, Cuxhaven) wurden mit 5 x 10⁶ KBE/ml des E.-coli-Stammes 6200 Serovar 078 (Prof. Hinz, Hannover) intrathorakal infiziert. Unmittelbar nach der Infektion erhielten jeweils Gruppen von 10 bis 20 Küken den Wirkstoff oral appliziert. Ausgetestet wurden 6 Verdünnungen einer geometrischen Verdünnungsreihe. Die Auswertung erfolgte durch Aufnahme der Mortalitätsrate 48 Stunden nach der Infektion. Die Wirksamkeit der betreffenden Verbindung wird als ED₉₀ in mg/kg angegeben.

Als Vergleich wurde die aus der EP-A 230 053 bekannte Verbindung 1-Cyclopropyl-6-fluor-1,4-dihydro-4-oxo-7-(3-vinyl-1,4-piperazin-1-yl)-3-chinoloncarbonsäure (Beispiel 102) herangezogen.

| | Bsp. 1 | Bsp. 102 aus EP-A 230 053 |
|---|---|---|
| ED₉₀ | 1,25 | 5 - 10 |

### Herstellung der Wirkstoffe

### Beispiel 1 8-Chlor-1-cyclopropyl-6-fluor-1,4-dihydro-4-oxo-7-(3-vinyl-1,4-piperazin-1-yl)-3-chinolincarbonsäure

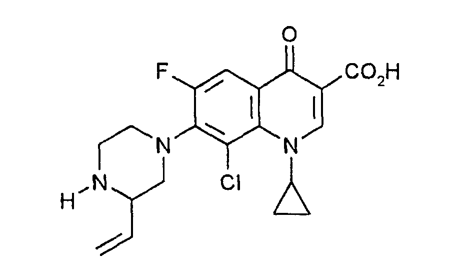

2,4 g 8-Chlor-1-cyclopropyl-6,7-difluor-1,4-dihydro-4-oxo-3-chinolincarbonsäure, 1,08 g 2-Vinylpiperazin und 1,12 g DABCO werden in einem Gemisch aus 12 ml Acetonitril und 4 ml Dimethylformamid 5 Stunden bei Rückflußtemperatur erhitzt. Das Reaktionsgemisch wird im Vakuum eingeengt und der Rückstand mit Wasser verrührt. Der erhaltene Feststoff wird abgesaugt, mit Wasser gewaschen und getrocknet. Man erhält 1,64 g Feststoff (52 % der Theorie) vom Schmelzpunkt 154 bis 156°C.

¹H-NMR (400 MHz, CDCl₃): δ = 0,95 (m,2H), 1,35 (m,2H), 3,15 (m,2H), 3,3 (m,2H), 3,4 (m,1H), 3,55 (m,1H), 4,35 (m, 1H), 5,2 (d,1H), 5,32 (d,1H), 5,8 - 5,9 (m,1H), 8,03 (d,1H), 8,9 (s,1H) ppm.

### Beispiel 2 8-Chlor-1-cyclopropyl-6-fluor-1,4-dihydro-4-oxo-7-(3-vinyl-1,4-piperazin-1-yl)-3-chinolincarbonsäure- Hydrochlorid

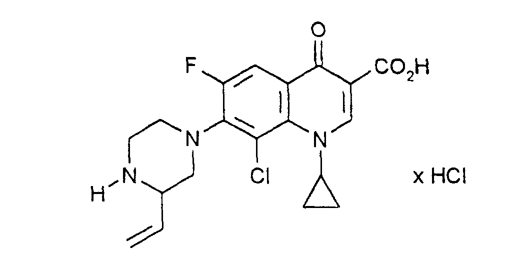

1 g 8-Chlor-1-cyclopropyl-6-fluor-1,4-dihydro-4-oxo-7-(3-vinyl-1,4-piperazin-1-yl)-3-chinolincarbonsäure werden in 3 ml konzentrierter wäßriger Salzsäure gelöst. Die Lösung wird im Vakuum zur Trockne eingeengt. Man erhält 1,09 g Feststoff vom Schmelzpunkt 299 bis 301°C.

### Beispiel 3 8-Chlor-1-cyclopropyl-7-[4-(2-ethoxycarbonyl-vinyl)-3-vinyl-1,4-piperazin-1-yl]-6-fluor-1,4-dihydro-4-oxo-3-chinolincarbonsäure

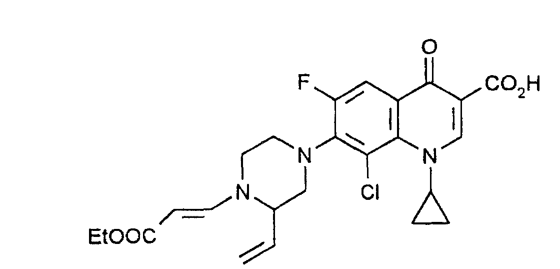

0,392 g 8-Chlor-1-cyclopropyl-6-fluor-1,4-dihydro-4-oxo-7-(3-vinyl-1,4-piperazin-1-yl)-3-chinolincarbonsäure und 0,98 g Propiolsäureethylester werden in 7 ml Ethylenglykolmonomethylether 1 Stunde unter Rückfluß erhitzt. Das Reaktionsgemisch wird im Vakuum eingeengt und der Rückstand in Methylenchlorid gelöst. Man schüttelt mit Wasser aus, trocknet die organische Phase über Natriumsulfat und engt ein. Der Rückstand wird an Kieselgel chromatographiert. Man erhält 0,3 g Feststoff (61 % der Theorie) vom Schmelzpunkt 128 bis 130°C (Zersetzung).

### Beispiel 4 5-Amino-1-cyclopropyl-6,8-difluor-1,4-dihydro-4-oxo-7-(3-vinyl-1,4-piperazin-1-yl)-chinolincarbonsäure

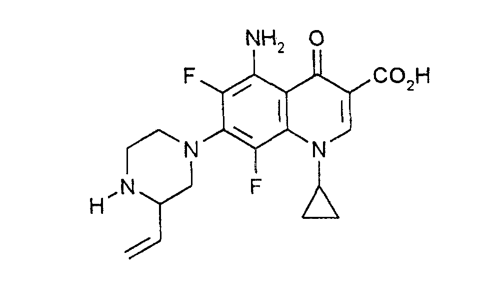

0,15 g 5-Amino-1-cyclopropyl-6,7,8-trifluor-1,4-dihydro-4-oxo-3-chinolincarbonsäure, 0,09 g 2-Vinylpiperazin und 0,09 g DABCO werden in einem Gemisch aus 2 ml Acetonitril und 1 ml Dimethylformamid 4 Stunden bei Rückfluß erhitzt. Das Reaktionsgemisch wird im Vakuum eingeengt und der Rückstand mit Wasser verrührt. Der erhaltene Feststoff wird abgesaugt, mit Wasser gewaschen und getrocknet. Man erhält 0,12 g Feststoff (62 % der Theorie) vom Schmelzpunkt 214 bis 216°C.

### Beispiel 5 1-Cyclopropyl-6-fluor-8-difluormethoxy-1,4-dihydro-4-oxo-7-(3-vinyl-1,4-piperazin-1-yl)-3-chinolincarbonsäure

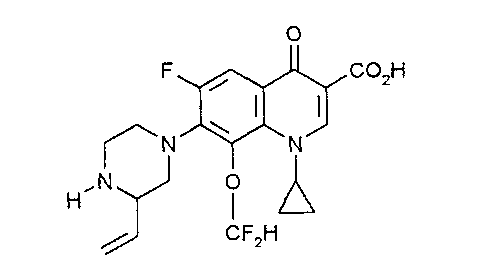

0,17 g 1-Cyclopropyl-6,7-difluor-8-difluormethoxy-1,4-dihydro-4-oxo-chinolincarbonsäure, 0,09 g 3-Vinylpiperazin und 0,067 g DABCO werden in einem Gemisch aus 1 ml Acetonitril und 0,5 ml Dimethylformamid 20 Stunden unter Rückfluß erhitzt. Das Reaktionsgemisch wird im Vakuum eingeengt und der Rückstand mit Wasser verrührt. Der erhaltene Feststoff wird abgesaugt, mit Wasser gewaschen und getrocknet. Man erhält 0,155 g Feststoff (73 % der Theorie) vom Schmelzpunkt 209°C.

### Beispiel 7 8-Cyan-1-cyclopropyl-6-fluor-7-(3'-vinyl-piperazin-1-yl)-1,4-dihydro-4-oxo-3-chinolincarbonsäure

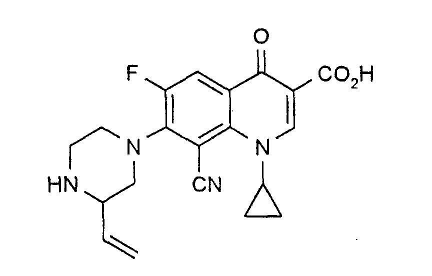

150 mg 7-Chlor-8-cyan-1-cyclopropyl-6-fluor-1,4-dihydro-4-oxo-3-chinolincarbonsäure werden mit 66 mg 2-Vinyl-piperazin und 0,136 ml Triethylamin in 5 ml Acetonitril 7 Stunden bei 40 bis 45°C gerührt. Alle flüchtigen Komponenten werden im Vakuum entfernt und der Rückstand aus Ethanol umkristallisiert.
Ausbeute: 160 mg (85 % der Theorie)
Schmelzpunkt: 265°C (Zersetzung)

### Beispiel 8 8-Chlor-1-cyclopropyl-6-fluor-1,4-dihydro-4-oxo-7-(3-vinyl-1,4-piperazin- 1-yl)-3-chinolincarbonsäuremethylester

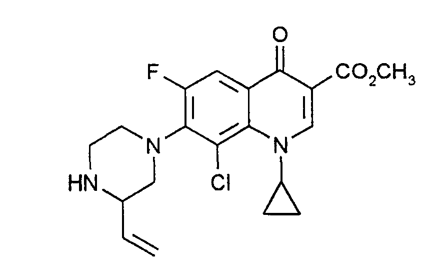

100 mg der Verbindung aus Beispiel 1 werden in einer Mischung aus 3 ml 2,2-Dimethoxypropan, 0,26 ml 33%iger Salzsäure und 1 ml mit HCl-Gas gesättigtem Methanol 24 Stunden bei Raumtemperatur gerührt. Das Reaktionsgemisch wird in 50 ml Wasser gegeben und dreimal mit je 15 ml Ether ausgeschüttelt. Die wäßrige Phase wird dann mit Sodalösung alkalisch gestellt und dreimal mit je 20 ml Chloroform ausgeschüttelt. Die organische Phase wird über Natriumsulfat getrocknet und eingeengt. Der Rückstand wird an Kieselgel chromatographiert (Essigester/Ethanol/Ammoniak 88:10:2) und durch Verrühren mit wenig Ether kristallisiert. Man erhält 42 mg Feststoff (41 % der Theorie) vom Schmelzpunkt 148°C.

### Beispiel 9

### 1-Cyclopropyl-6-fluor-1,4-dihydro-4-oxo-5-vinyl-7-(3-vinyl-1,3-piperazin-1-yl)-3-chinolincarbonsäure

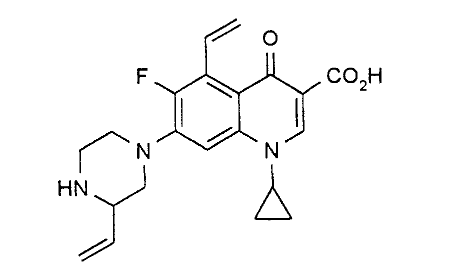

0,29 g 1-Cyclopropyl-6,7-dilfuor-1,4-dihydro-4-oxo-5-vinyl-3-chinolincarbonsäure, 0,28 g 2-Vinyl-piperazin-dihydrochlorid und 0,34 g DABCO werden in einem Gemisch aus 2 ml Autonitril und 1 ml Dimethylformamid 4 Std. unter Rückfluß erhitzt. Das Reaktionsgemisch wird im Vakuum eingeengt und der Rückstand mit Waser verrührt. Der resultierende Feststoff wird abgesaugt, mit Wasser gewaschen und getrocknet. Man erhält 0,20 g beigen Farbstoff (52 % der Theorie) vom Schmelzpunkt 170°C (Zersetzung).

### Beispiel 10

### 1-Cyclopropyl-6-fluor-1,4-dihydro-5-methyl-4-oxo-7-(3-vinyl-1,4-piperazin-1-yl)-3-chinolincarbonsäure

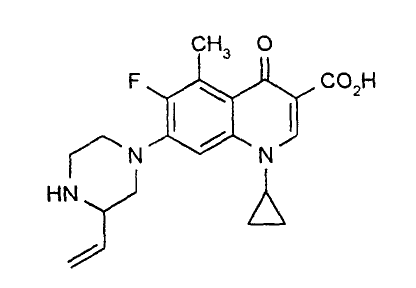

0,28 g 1-Cyclopropyl-6,7-difluor-1,4-dihydro-5-methyl-4-oxo-3-chinolincarbonsäure, 0,28 g 2-Vinylpiperazin-dihydrochlorid und 0,5 g DABCO werden in einem Gemisch aus 3 ml Acetonitril und 1 ml Dimethylformamid 5 Stunden unter Rückfluß erhitzt. Das Reaktionsgemisch wird im Vakuum weitgehend eingeengt und der Rückstand mit Wasser verrührt. Nach Filtration wird das Filtrat mehrmals mit Chloroform extrahiert. Die vereinigten organischen Phasen werden über Natriumsulfat getrocknet und im Vakuum eingeengt. Der erhaltene Feststoff wird im Vakuum getrocknet. Ausbeute: 0,243 g beiger Feststoff (65 % der Theorie) vom Schmelzpunkt 110-112°C.

### Vergleichsverbindung

### 1-Cyclopropyl-6-fluor-1,4-dihydro-4-oxo-7-(3-vinyl-1,4-piperazin-1-yl)-3-chinolincarbonsäure

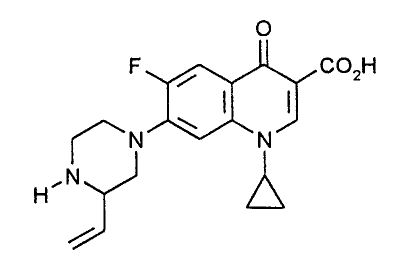

0,53 g 1-Cyclopropyl-6,7-fluor-1,4-dihydro-4-oxo-3-chinolincarbonsäure, 0,56 g 2-Vinylpiperazin-Dihydrochlorid und 0,67 g DABCO werden in einem Gemisch aus 4 ml Acetonitril und 2 ml Dimethylformamid 4 Stunden unter Rückfluß erhitzt. Das Reaktionsgemisch wird filtriert, das Filtrat im Vakuum eingeengt und der Rückstand mit Wasser verrührt. Der so erhaltene Feststoff wird abgesaugt, mit Wasser gewaschen und getrocknet. Man erhält 0,41 g Feststoff (57 % der Theorie) vom Schmelzpunkt > 295°C.

## Patentansprüche

1. Verbindungen der allgemeinen Formel (I) 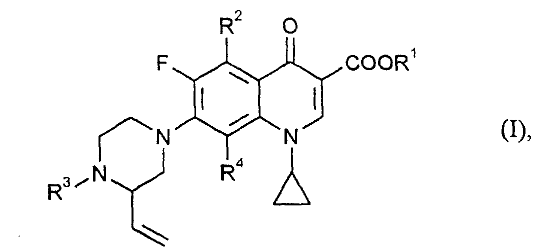 in welcher
R¹ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
R² für Wasserstoff oder Fluor steht, wenn gleichzeitig R⁴ ungleich Wasserstoff oder Fluor ist, oder für Amino, Methyl oder Vinyl steht,
R³ für Wasserstoff, Benzyl, Alkyl mit 1 bis 3 Kohlenstoffatomen, Reste der Strukturen -CH=CH-COOR⁵, -CH₂CH₂-COOR⁵, -CH₂CH₂CN, -CH₂CH₂COCH₃, -CH₂COCH₃, in denen R⁵ für Methyl oder Ethyl steht, oder einen Rest der allgemeinen Struktur R⁶-(NH-CHR⁷-CO)ₙ- steht, in der R⁶ für Wasserstoff, Alkyl mit 1 bis 3 Kohlenstoffatomen oder den Rest -COO-tert.-Butyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht und n = 1 oder 2 ist,
und
R⁴ für Wasserstoff oder Fluor steht, wenn R² für Amino, Methyl oder Vinyl steht, oder für Chlor, Methoxy, Difluormethoxy, CN oder Ethinyl steht.

2. Verbindungen der Formel (I) gemäß Anspruch 1, in welcher
R¹ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
R² für Wasserstoff oder Fluor steht, wenn gleichzeitig R⁴ ungleich Wasserstoff oder Fluor ist, oder für Amino oder Methyl steht,
R³ für Wasserstoff, Alkyl mit 1 bis 3 Kohlenstoffatomen, Reste der Strukturen -CH=CH-COOR⁵, -CH₂CH₂-COOR⁵, -CH₂CH₂CN, -CH₂COCH₃, R⁶-(NH-CHR⁷-CO)ₙ- steht, in der R⁶ für Wasserstoff, Alkyl mit 1 bis 3 Kohlenstoffatomen oder den Rest -COO-tert.-Butyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht und n = 1 oder 2 ist,
und
R⁴ für Wasserstoff oder Fluor steht, wenn R² für Amino oder Methyl steht, oder für Chlor, Methoxy, Difluormethoxy oder CN steht.

3. Verbindungen der Formel (I) gemäß Anspruch 1, in welcher
R¹ für Wasserstoff, Methyl oder Ethyl steht,
R² für Wasserstoff oder Fluor steht, wenn gleichzeitig R⁴ ungleich Wasserstoff oder Fluor ist, oder für Amino steht,
R³ für Wasserstoff, Methyl, Reste der Strukturen -CH=CH-COOR⁵, -CH₂CH₂-CN oder -CH₂COCH₃, in denen R⁵ für Methyl oder Ethyl steht, oder einen Rest der allgemeinen Struktur R⁶-(NH-CHR⁷-CO)ₙ- steht, in der R⁶ für Wasserstoff oder Methyl steht, R⁷ für Wasserstoff, Alkyl mit 1 bis 4 Kohlenstoffatomen, Hydroxyalkyl mit 1 oder 2 Kohlenstoffatomen, CH₂-Phenyl, Aminoalkyl oder Thioalkyl mit 1 oder 2 Kohlenstoffatomen steht, und n = 1 oder 2 ist,
und
R⁴ für Wasserstoff oder Fluor steht, wenn R² für Amino steht, oder für Chlor, Methoxy, Difluormethoxy oder CN steht.

4. Verfahren zur Herstellung der Verbindungen der Formel (I) gemäß Anspruch 1, **dadurch gekennzeichnet, daß** man Verbindungen der Formel (II) 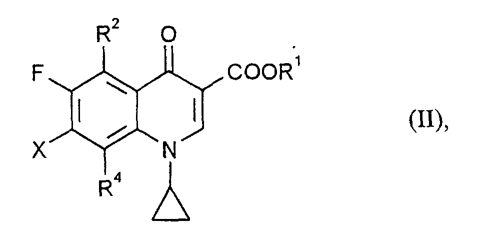 in welcher
R¹, R² und R⁴ die in Anspruch 1 angegebene Bedeutung haben und
X für Fluor oder Chlor steht,
mit Verbindungen der Formel (III) 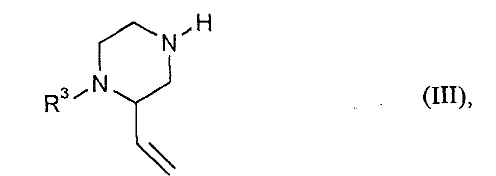 in welcher R³ die in Anspruch 1 angegebene Bedeutung hat, gegebenenfalls in Gegenwart von Säurefängern umsetzt.

5. 8-Chlor-1-cyclopropyl-6-fluor-1,4-dihydro-4-oxo-7-(3-vinyl-1,4-piperazin-1-yl)-3-chinolincarbonsäure und deren Ester gemäß Anspruch 1.

6. 5-Amino-1-cyclopropyl-6,8-difluor-1,4-dihydro-4-oxo-7-(3-vinyl-1,4-piperazin-1-yl)-3-chinolincarbonsäure und deren Ester gemäß Anspruch 1.

7. 1-Cyclopropyl-6-fluor-8-difluormethoxy-1,4-dihydro-4-oxo-7-(3 -vinyl-1,4-piperazin-1-yl)-3-chinolincarbonsäure und deren Ester gemäß Anspruch 1.

8. Arzneimittel enthaltende Verbindungen der Formel (I) gemäß Anspruch 1.

9. Verwendung von Verbindungen der Formel (I) gemäß Anspruch 1 zur Herstellung von Arzneimitteln.

10. Verwendung von Verbindungen der Formel (I) gemäß Anspruch 1 zur Herstellung von antibakteriellen Mitteln.

## Claims

1. Compounds of the general formula (I) 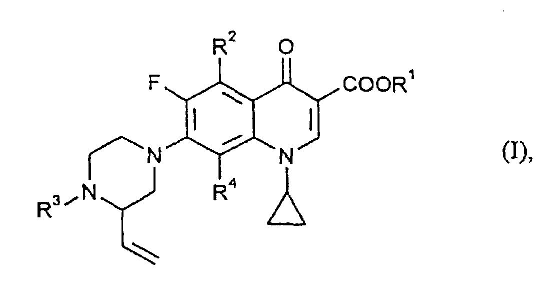 in which
R¹ represents hydrogen, optionally hydroxyl-, methoxy-, amino-, methylamino- or dimethylamino-substituted alkyl having I to 4 carbon atoms or (5-methyl-2-oxo-1,3-dioxol-4-yl)-methyl,
R² represents hydrogen or fluorine if R⁴ is simultaneously unequal to hydrogen or fluorine, or represents amino, methyl or vinyl,
R³ represents hydrogen, benzyl, alkyl having 1 to 3 carbon atoms, radicals of the structures -CH=CH-COOR⁵, -CH₂CH₂-COOR⁵, -CH₂CH₂CN, -CH₂CH₂COCH₃, -CH₂COCH₃, in which R⁵ represents methyl or ethyl, or a radical of the general structure R⁶-(NH-CHR⁷-CO)ₙ-, in which R⁶ represents hydrogen, alkyl having 1 to 3 carbon atoms or the radical -COO-tert-butyl, R⁷ represents hydrogen, alkyl having 1 to 4 carbon atoms, hydroxyalkyl having 1 or 2 carbon atoms, CH₂-phenyl, aminoalkyl or thioalkyl having 1 or 2 carbon atoms and n = 1 or 2,
and
R⁴ represents hydrogen or fluorine if R² represents amino, methyl or vinyl, or represents chlorine, methoxy, difluoromethoxy, CN or ethinyl.

2. Compounds of the formula (I) according to Claim 1, in which
R¹ represents hydrogen, optionally hydroxyl-, methoxy-, amino-, methylamino- or dimethylamino-substituted alkyl having 1 to 4 carbon atoms or (5-methyl-2-oxo-1,3-dioxol-4-yl)-methyl,
R² represents hydrogen or fluorine when R⁴ is simultaneously unequal to hydrogen or fluorine, or represents amino or methyl,
R³ represents hydrogen, alkyl having 1 to 3 carbon atoms, radicals of the structures -CH=CH-COOR⁵, -CH₂CH₂-COOR⁵, -CH₂CH₂CN, -CH₂COCH₃, in which R⁵ represents methyl or ethyl, or a radical of the general structure, R⁶-(NH-CHR⁷-CO)ₙ- in which R⁶ represents hydrogen, alkyl having 1 to 3 carbon atoms or the radical -COO-tert-butyl, R⁷ represents hydrogen, alkyl having 1 to 4 carbon atoms, hydroxyalkyl having 1 or 2 carbon atoms, CH₂-phenyl, aminoalkyl or thioalkyl having 1 or 2 carbon atoms and n = 1 or 2,
and
R⁴ represents hydrogen or fluorine if R² represents amino or methyl, or represents chlorine, methoxy, difluoromethoxy or CN.

3. Compounds of the formula (I) according to Claim 1, in which
R¹ represents hydrogen, methyl or ethyl,
R² represents hydrogen or fluorine if R⁴ is simultaneously unequal to hydrogen or fluorine, or represents amino,
R³ represents hydrogen, methyl, radicals of the structures -CH=CH-COOR⁵, -CH₂CH₂-CN or -CH₂COCH₃, in which R⁵ represents methyl or ethyl, or a radical of the general structure R⁶-(NH-CHR⁷-CO)ₙ-, in which R⁶ represents hydrogen or methyl, R⁷ represents hydrogen, alkyl having 1 to 4 carbon atoms, hydroxyalkyl having 1 or 2 carbon atoms, CH₂-phenyl, aminoalkyl or thioalkyl having 1 or 2 carbon atoms, and n = 1 or 2,
and
R⁴ represents hydrogen or fluorine if R² represents amino, or represents chlorine, methoxy, difluoromethoxy or CN.

4. Process for the preparation of the compounds of the formula (I) according to Claim 1, **characterized in that** compounds of the formula (II) 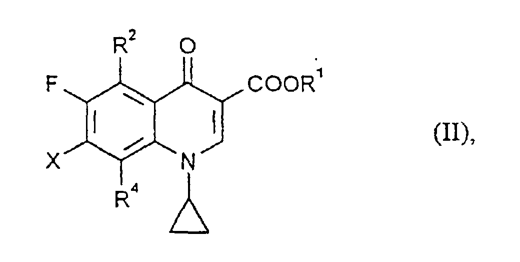 in which
R¹, R² and R⁴ have the meaning indicated in Claim 1 and
X represents fluorine or chlorine,
are reacted with compounds of the formula (III) 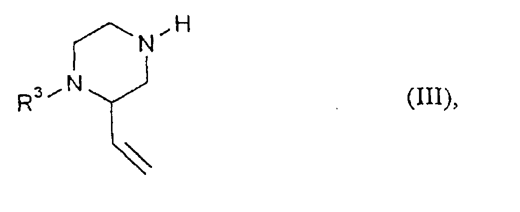 in which R³ has the meaning indicated in Claim 1, if appropriate in the presence of acid scavengers.

5. 8-Chloro-1-cyclopropyl-6-fluoro-1,4-dihydro-4-oxo-7-(3-vinyl-1,4-piperazin-1-yl)-3-quinolinecarboxylic acid and its esters according to Claim 1.

6. 5-Amino-1-cyclopropyl-6,8-difluoro-1,4-dihydro-4-oxo-7-(3-vinyl-1,4-piperazin-1-yl)-3-quinolinecarboxylic acid and its esters according to Claim 1.

7. 1-Cyclopropyl-6-fluoro-8-difluoromethoxy-1,4-dihydro-4-oxo-7-(3-vinyl-1,4-piperazin-1-yl)-3-quinolinecarboxylic acid and its esters according to Claim 1.

8. Medicaments comprising compounds of the formula (I) according to Claim 1.

9. Use of compounds of the formula (I) according to Claim 1 for the production of medicaments.

10. Use of compounds of the formula (I) according to Claim 1 for the production of antibacterial compositions.

## Revendications

1. Composés de formule générale (I) : 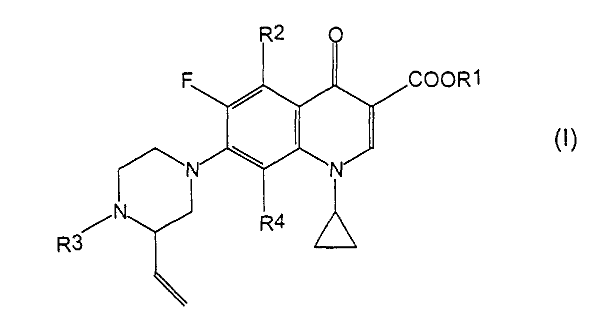 dans laquelle :
R¹ représente l'atome d'hydrogène, un radical alcoyle ayant 1 à 4 atomes de carbone, éventuellement substitué par le radical hydroxy, méthoxy, amino, méthylamino ou diméthylamino, ou le radical (5-méthyl-2-oxo-1,3-dioxol-4-yl)méthyle,
R² représente l'atome d'hydrogène ou de fluor, lorsque simultanément R⁴ est différent de l'atome d'hydrogène ou de fluor, ou il représente le radical amino, méthyle ou vinyle,
R³ représente l'atome d'hydrogène, le radical benzyle, un radical alcoyle ayant 1 à 3 atomes de carbone, les restes de structures -CH=CH-COOR⁵, -CH₂CH₂-COOR⁵, -CH₂CH₂-CN, -CH₂CH₂-COCH₃, -CH₂-COCH₃, dans lesquelles R⁵ représente le radical méthyle ou éthyle, ou un reste de structure générale R⁶-(NH-CHR⁷-CO)ₙ-, dans laquelle R⁶ représente l'atome d'hydrogène, un radical alcoyle ayant 1 à 3 atomes de carbone ou le reste -COO-t-butyle, R⁷ représente l'atome d'hydrogène, un radical alcoyle ayant 1 à 4 atomes de carbone, un radical hydroxyalcoyle ayant 1 ou 2 atomes de carbone, le radical -CH₂-phényle, un radical aminoalcoyle ou thioalcoyle ayant 1 ou 2 atomes de carbone, et n est 1 ou 2, et
R⁴ représente l'atome d'hydrogène ou de fluor, lorsque R² représente le radical amino, méthyle ou vinyle, ou il représente l'atome de chlore, le radical méthoxy, difluorométhoxy, CN ou éthynyle.

2. Composés de formule (I) suivant la revendication 1, dans laquelle :
R¹ représente l'atome d'hydrogène, un radical alcoyle ayant 1 à 4 atomes de carbone, éventuellement substitué par le radical hydroxy, méthoxy, amino, méthylamino ou diméthylamino, ou le radical (5-méthyl-2-oxo-1,3-dioxol-4-yl)méthyle,
R² représente l'atome d'hydrogène ou de fluor, lorsque simultanément R⁴ est différent de l'atome d'hydrogène ou de fluor, ou il représente le radical amino ou méthyle,
R³ représente l'atome d'hydrogène, un radical alcoyle ayant 1 à 3 atomes de carbone, les restes de structures -CH=CH-COOR⁵, -CH₂CH₂-COOR⁵, -CH₂CH₂-CN, -CH₂-COCH₃, R⁶-(NH-CHR⁷-CO)ₙ-, dans lesquelles R⁶ représente l'atome d'hydrogène, un radical alcoyle ayant 1 à 3 atomes de carbone ou le reste -COO-t-butyle, R⁷ représente l'atome d'hydrogène, un radical alcoyle ayant 1 à 4 atomes de carbone, un radical hydroxyalcoyle ayant 1 ou 2 atomes de carbone, le radical -CH₂-phényle, un radical aminoalcoyle ou thioalcoyle ayant 1 ou 2 atomes de carbone, et n est 1 ou 2, et
R⁴ représente l'atome d'hydrogène ou de fluor, lorsque R² représente le radical amino ou méthyle, ou il représente l'atome de chlore, le radical méthoxy, difluorométhoxy ou CN.

3. Composés de formule (I) suivant la revendication 1, dans laquelle :
R¹ représente l'atome d'hydrogène, le radical méthyle ou éthyle ;
R² représente l'atome d'hydrogène ou de fluor, lorsque simultanément R⁴ est différent de l'atome d'hydrogène ou de fluor, ou il représente le radical amino ;
R³ représente l'atome d'hydrogène, le radical méthyle, les restes de structures -CH=CH-COOR⁵, -CH₂CH₂-CN, -CH₂-COCH₃, dans lesquelles R⁵ représente le radical méthyle ou éthyle, ou R³ représente un reste de structure générale R⁶-(NH-CHR⁷-CO)ₙ-, dans laquelle R⁶ représente l'atome d'hydrogène ou le radical méthyle, R⁷ représente l'atome d'hydrogène, un radical alcoyle ayant 1 à 4 atomes de carbone, un radical hydroxyalcoyle ayant 1 ou 2 atomes de carbone, le radical -CH₂-phényle, un radical aminoalcoyle ou thioalcoyle ayant 1 ou 2 atomes de carbone, et n est 1 ou 2, et
R⁴ représente l'atome d'hydrogène ou de fluor, lorsque R² représente le radical amino, ou il représente l'atome de chlore, le radical méthoxy, difluorométhoxy ou CN.

4. Procédé de préparation des composés de formule (I) suivant la revendication 1, **caractérisé en ce que** l'on fait réagir les composés de formule (II) : 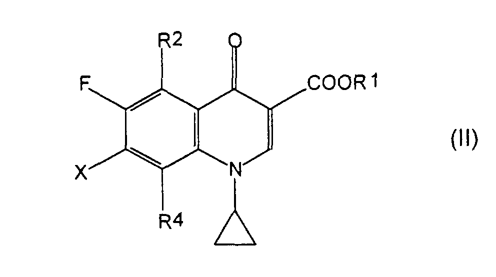 dans laquelle :
R¹, R² et R⁴ ont la signification indiquée à la revendication 1, et
X représente l'atome de fluor ou de chlore,
avec des composés de formule (III) : 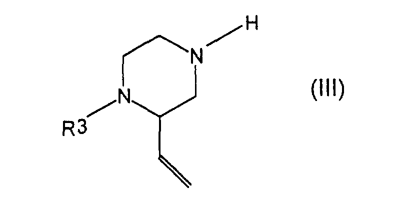 dans laquelle R³ a la signification indiquée à la revendication 1, le cas échéant en présence de capteurs d'acide.

5. Acide 8-chloro-1-cyclopropyl-6-fluoro-1,4-dihydro-4-oxo-7-(3-vinyl-1,4-pipérazin-1-yl)-3-quinoléinecarboxylique et ses esters suivant la revendication 1.

6. Acide 5-amino-1-cyclopropyl-6,8-difluoro-1,4-dihydro-4-oxo-7-(3-vinyl-1,4-pipérazin-1-yl)-3-quinoléinecarboxylique et ses esters suivant la revendication 1.

7. Acide 1-cyclopropyl-6-fluoro-8-difluoro-méthoxy-1,4-dihydro-4-oxo-7-(3-vinyl-1,4-pipérazin-1-yl)-3-quinoléinecarboxylique et ses esters suivant la revendication 1.

8. Agent pharmaceutique contenant des composés de formule (I) suivant la revendication 1.

9. Utilisation des composés de formule (I) suivant la revendication 1, pour préparer des agents pharmaceutiques.

10. Utilisation des composés de formule (I) suivant la revendication 1, pour préparer des agents antibactériens.
